(19) Europäisches Patentamt
European Patent Office
Office européen des brevets

(11) Veröffentlichungsnummer: **0 187 285**
**B1**

(12) **EUROPÄISCHE PATENTSCHRIFT**

(45) Veröffentlichungstag der Patentschrift: **06.09.89**

(51) Int. Cl.⁴: **C 07 C 109/04**, C 07 C 147/12, C 07 C 149/24 // C07D231/40, A01N43/56

(21) Anmeldenummer: **85115641.4**

(22) Anmeldetag: **09.12.85**

(54) **Verfahren zur Herstellung von substituierten Phenylhydrazinen.**

(30) Priorität: **22.12.84 DE 3447211**

(43) Veröffentlichungstag der Anmeldung: **16.07.86 Patentblatt 86/29**

(45) Bekanntmachung des Hinweises auf die Patenterteilung: **06.09.89 Patentblatt 89/36**

(84) Benannte Vertragsstaaten: **AT BE CH DE FR GB IT LI NL**

(56) Entgegenhaltungen:
**EP-A- 0 138 149**
**EP-A- 0 191 282**
**DE-A- 2 015 405**

**J. LIEBIGS: "Annalen der Chemie", Band 716, 1968, Seiten 47-60, Verlag Chemie GmbH, Weinheim/Bergstr., DE; G. BECK et al.: "Nucleophile Substitution an chlorierten Mono- und Dicyan-benzolen"**

(73) Patentinhaber: **BAYER AG, D-5090 Leverkusen 1 Bayerwerk (DE)**

(72) Erfinder: **Gallenkamp, Bernd, Dr., Claudiusweg 5, D-5600 Wuppertal 1 (DE)**
Erfinder: **Schallner, Otto, Dr., Noldeweg 22, D-4019 Monheim (DE)**
Erfinder: **Klauke, Erich, Dr., Eichendorffweg 8, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 187 285 B1

**Beschreibung**

Die Erfindung betrifft ein neues Verfahren zur Herstellung von substituierten Phenylhydrazinen, die als Zwischenprodukte für die Synthese von herbiziden Wirkstoffen verwendet werden können.

Es ist bereits bekannt, daß man substituierte Phenylhydrazine erhält, wenn man substituierte Aniline zunächst mit Natriumnitrit in Gegenwart einer Säure diazotiert und dann in einer zweiten Stufe beispielsweise mit Zinn-II-chlorid ebenfalls in Gegenwart einer Säure reduziert (vgl. z.B. Houben-Weyl, «Methoden der organischen Chemie», Band X, 2; Seite 203, Thieme Verlag Stuttgart 1967).

Der Nachteil dieses Verfahrens besteht in oft nicht zufriedenstellender Ausbeute und Reinheit der so erhältlichen Reaktionsprodukte, eine Tatsache, die einerseits begründet ist in den allgemeinen Nachteilen einer mehrstufigen Reaktionsführung, andererseits auch auf spezielle Probleme in beiden Stufen zurückzuführen ist. So gelingt beispielsweise die Herstellung der Diazoniumsalze (1. Stufe), wenn man als Ausgangsverbindungen Aniline mit hydrophoben Substituenten verwendet, in dem erforderlichen wäßrigen Medium oft nur schlecht. Eine weitere Schwierigkeit der Diazotierung liegt darin, daß Nebenreaktionen wie beispielsweise die Azokupplung möglich sind und auch auftreten. Auch die Reduktion der Diazoniumsalze (2. Stufe) gelingt häufig nicht vollständig, was ebenfalls zur Verunreinigung und Ausbeuteminderung beiträgt.

Es wurde gefunden, daß man substituierte Phenylhydrazine der Formel (I)

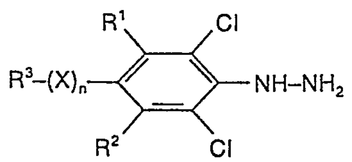
(I)

in welcher

$R^1$ und $R^2$ für Wasserstoff oder Chlor stehen,

$R^3$ für Halogenalkyl steht,

X für Sauerstoff, Schwefel, Sulfinyl oder Sulfonyl steht und

n für eine Zahl 0 oder 1 steht,

erhält, wenn man substituierte Chlorbenzole der Formel (II)

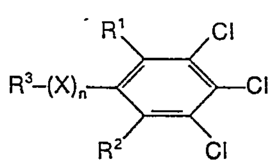
(II)

in welcher

$R^1$, $R^2$, $R^3$, X und n die oben angegebenen Bedeutungen haben,

mit Hydrazin oder Hydrazinhydrat in Gegenwart eines Verdünnungsmittels und gegebenenfalls bei erhöhtem Druck bei Temperaturen zwischen 60 °C und 160 °C umsetzt.

Es ist als ausgesprochen überraschend anzusehen, daß man mit Hilfe des erfindungsgemäßen Verfahrens die gewünschten substituierten Phenylhydrazine in hohen Ausbeuten und guter Reinheit erhält, da nach dem Stand der Technik nicht zu erwarten war, daß bei den mindestens drei Chloratomen, die im Ausgangsprodukt der Formel (II) für die erfindungsgemäße Substitutionsreaktion zur Verfügung stehen, selektiv nur ein Produkt entsteht.

Das erfindungsgemäße Verfahren weist eine Reihe von Vorteilen auf. Dazu gehört beispielsweise die einstufige Reaktionsführung in einem organischen Lösungsmittel, wodurch Löslichkeitsprobleme bei der Verwendung von hydrophoben Ausgangsstoffen vermieden werden. Ebenfalls vermeidet man die aufwendige Isolierung von Zwischenprodukten. Ein weiterer Vorteil des erfindungsgemäßen Verfahrens ist die leichte Zugänglichkeit der als Ausgangsverbindungen in Frage kommenden substituierten Chlorbenzole der Formel (II).

Mit Hilfe des erfindungsgemäßen Verfahrens erhält man vorzugsweise solche substituierten Phenylhydrazine der Formel (I), bei welchen

$R^1$ und $R^2$ für Wasserstoff oder Chlor stehen,

X für Sauerstoff, Schwefel, Sulfinyl oder Sulfonyl steht,

$R^3$ für geradkettiges oder verzweigtes Halogenalkyl mit 1 bis 4 Kohlenstoffatomen und 1 bis 9 gleichen oder verschiedenen Halogenatomen steht und

n für eine Zahl 0 oder 1 steht.

Besonders bevorzugt erhält man Verbindungen der Formel (I), bei welchen

$R^1$ und $R^2$ für Wasserstoff oder Chlor stehen,

$R^3$ für Difluormethyl, Trifluormethyl, Trichlormethyl, Dichlorfluormethyl, Difluorchlormethyl, Trifluorethyl, Tetrafluorethyl, Pentafluorethyl, Tetrafluorchlorethyl, Trifluorchlorethyl, Trifluordichlorethyl, Difluortrichlorethyl, Difluortrichlorethyl, Difluordichlorethyl, Tetrachlorfluorethyl oder Pentachlorethyl stehen,

X für Schwefel, Sulfinyl oder Sulfonyl steht und

n für eine Zahl 0 oder 1 steht.

Verwendet man beispielsweise 1,2,3-Trichlor-5-trifluormethylbenzol und Hydrazinhydrat als Ausgangsstoffe, so läßt sich der Reaktionsablauf des erfindungsgemäßen Verfahrens durch das folgende Formelschema darstellen:

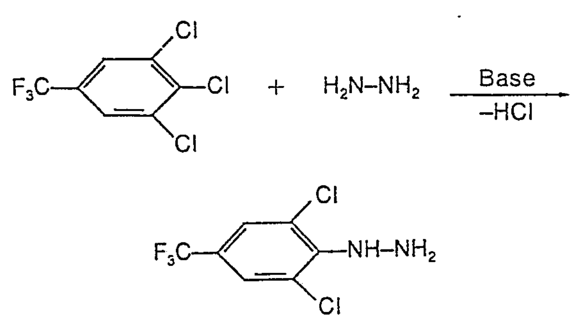


Die zur Durchführung des erfindungsgemäßen Verfahrens als Ausgangsstoffe benötigten substituierten Chlorbenzole sind durch die Formel (II) allgemein definiert.

Bevorzugt sind Verbindungen der Formel (II), bei welchen $R^1$, $R^2$, $R^3$, X und n für diejenigen Reste stehen, die bereits im Zusammenhang mit der Beschreibung der Endprodukte der Formel (I) als bevorzugt für diese Substituenten und Indices genannt wurden.

Die substituierten Chlorbenzole der Formel (II) sind bekannt (vgl. z.B. DE-OS 2 644 641; DE-OS 2 333 848; JP 49/2108; J. Fluorine Chem. 9, 113–126 (1977); Ind. Eng. Chem. 39, 378–380 (1947)) oder lassen sich nach prinzipiell bekannten Verfahren in analoger Weise herstellen. Das erfindungsgemäße Verfahren kann gegebenenfalls in Gegenwart eines geeigneten Verdünnungsmittels durchgeführt werden. Vorzugsweise arbeitet man bei dem erfindungsgemäßen Verfahren mit Verdünnungsmitteln.

Als Verdünnungsmittel kommen praktisch alle inerten organischen Lösungsmittel in Frage. Hierzu gehören insbesondere polare oder dipolar aprotische Lösungsmittel wie Alkohole, beispielsweise Methanol, Ethanol, n- oder i-Propanol, Ethylenglykol, oder Ether, wie beispielsweise Dioxan, Dimethoxyethan, Diethylenglykoldimethylether, oder Sulfoxide, wie beispielsweise Dimethylsulfoxid. Besonders bevorzugte Verdünnungsmittel sind Dioxan und n-Propanol.

Besonders bevorzugt als Verdünnungsmittel sind auch schwach basische Lösungsmittel, wie beispielsweise Pyridin.

Das erfindungsgemäße Verfahren wird im allgemeinen bei Temperaturen zwischen +60 °C und +160 °C durchgeführt. Bevorzugt ist der Temperaturbereich zwischen +80 °C und +140 °C, insbesondere zwischen +100 °C und 120 °C. Die Umsetzungen werden im allgemeinen bei Normaldruck durchgeführt. Je nach Siedepunkt des verwendeten Verdünnungsmittels kann es jedoch auch von Vorteil sein, den zum Erreichen der erforderlichen Reaktionstemperatur nötigen Druck anzulegen. In diesen Fällen arbeitet man im allgemeinen im Druckbereich von 1 bis 30 bar, bevorzugt von 1 bis 20 bar.

Zur Durchführung des erfindungsgemäßen Verfahrens setzt man im allgemeinen pro Mol an substituiertem Chlorbenzol der Formel (II) 1,0 bis 10,0 Mol, vorzugsweise 2,0 bis 8,0 Mol, insbesondere 3,0 bis 7,0 Mol an Hydrazin oder Hydrazinhydrat ein.

Die Reaktion wird so durchgeführt, daß man die Reaktionspartner in dem entsprechenden Verdünnungsmittel gelöst für 8 bis 60 Stunden auf die erforderliche Reaktionstemperatur erhitzt (der Reaktionsverlauf kann dabei z.B. gaschromatographisch verfolgt werden), wobei bei Verwendung von niedrig siedenden Verdünnungsmitteln gegebenenfalls unter Druck gearbeitet werden muß, damit die erforderliche Reaktionstemperatur erreicht wird.

Zur Aufarbeitung destilliert man das Lösungsmittel ab, nimmt den Rückstand in Wasser auf, stellt die Mischung mit wäßriger Natriumhydroxid-Lösung auf pH 10 ein und extrahiert das Produkt mit einem mit Wasser nicht mischbaren Lösungsmittel. Die vereinigten organischen Phasen werden gegebenenfalls nochmals mit konzentrierter wäßriger Natriumchlorid-Lösung gewaschen, getrocknet und im Vakuum eingeengt.

Die so erhaltenen Rohprodukte der Formel (I) lassen sich entweder destillativ reinigen oder aus einem geeigneten Lösungsmittel, wie z.B. n-Hexan kristallisieren.

Die substituierten Phenylhydrazine der Formel (I) sind wertvolle Zwischenprodukte und eignen sich beispielsweise zur Synthese von herbizid wirksamen 5-Acylamino-1-phenylpyrazolen, die beispielsweise in den vorgängigen noch nicht veröffentlichten Patentanmeldungen DE-P 3 337 543 vom 15.10.1983 oder DE-P 3 402 308 vom 24.1.1984 oder DE-P 3 420 985.9 vom 6.6.1984 beschrieben sind.

So erhält man neue 5-Aminopyrazole der Formel (III)

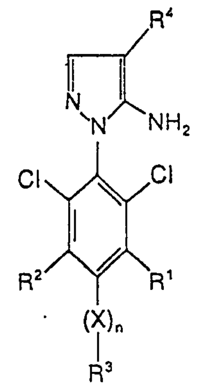

(III)

in welcher

$R^4$ für Cyano oder für Alkylaminocarbonyl steht und

$R^1$, $R^2$, $R^3$, X und n die oben angegebene Bedeutung haben,

wenn man Acrylnitril-Derivate der Formel (IV)

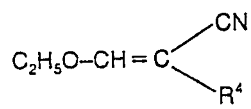

(IV)

in welcher

$R^4$ die oben angegebene Bedeutung hat,

mit Phenylhydrazinen der Formel (I)

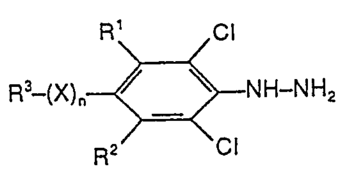

(I)

in welcher

$R^1$, $R^2$, $R^3$, X und n die oben angegebene Bedeutung haben,

entweder zunächst in einer ersten Stufe, gegebenenfalls in Gegenwart eines Verdünnungsmittels, wie beispielsweise Eisessig oder Ethanol, sowie gegebenenfalls in Gegenwart eines Reaktionshilfsmittels, wie beispielsweise Natriumacetat, bei Temperaturen zwischen −20 °C und +20 °C umsetzt zu den Phenylhydrazin-Derivaten der Formel (V)

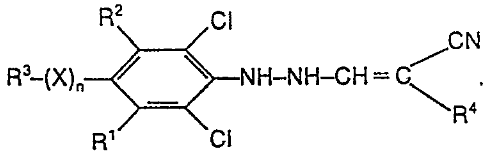

. (V)

in welcher

$R^1$, $R^2$, $R^3$, $R^4$, X und n die oben angegebene Bedeutung haben,

und diese in einer zweiten Stufe, gegebenenfalls in Gegenwart eines Verdünnungsmittels wie beispielsweise Ethylenglykolmonoethylether, bei Temperaturen zwischen +50 °C und +150 °C cyclisiert, oder direkt in einem Reaktionsschritt ohne Isolierung der Zwischenstufe der Formel (V), gegebenenfalls in Gegenwart eines Verdünnungsmittels wie beispielsweise Ethylenglykolmonoethylether oder Ethanol bei Temperaturen zwischen +50 °C und +150 °C cyclisiert.

Die Acrylnitril-Derivate der Formel (IV) sind bekannt (vgl. EP 34 945 oder DE-OS 3 129 429).

Man erhält neue herbizid wirksame substituierte 5-Acylamino-1-phenylpyrazole der Formel (VI)

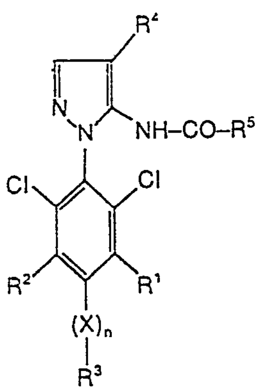

(VI)

in welcher

$R^1$, $R^2$, $R^3$, $R^4$, X und n die oben angegebene Bedeutung haben und

$R^5$ für Wasserstoff, für Alkyl, Alkenyl, Alkinyl, für gegebenenfalls substituiertes Cycloalkyl, für Alkoxyalkyl, Alkylthioalkyl, Halogenalkyl oder für gegebenenfalls substituiertes Aryl steht,

wenn man 5-Aminopyrazole der Formel (III)

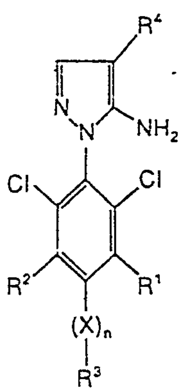

(III)

in welcher

$R^1$, $R^2$, $R^3$, $R^4$, X und n die oben angegebene Bedeutung haben,

mit Acylierungsmitteln der Formel (VII)

$$R^5-CO-A \quad (VII)$$

in welcher

$R^5$ die oben angegebene Bedeutung hat und

A für eine aktivierende Abgangsgruppe steht wie beispielsweise Halogen oder für einen Rest $R^5-CO-O$ steht, wobei $R^5$ die oben angegebene Bedeutung hat,

gegebenenfalls in Gegenwart eines Verdünnungsmittels wie z. B. Chloroform und gegebenenfalls in Gegenwart eines Säurebindemittels wie z. B. Triethylamin bei Temperaturen zwischen −20 und +150 °C umsetzt.

Zur Durchführung dieses Verfahrens setzt man pro Mol 5-Aminopyrazol der Formel (III) im allgemeinen 1 bis 20 Mol, vorzugsweise 1 bis 15 Mol an Acylierungsmittel der Formel (VII) und im allgemeinen 1 bis 3 Mol, vorzugsweise 1 bis 2 Mol an Säurebindemittel ein. Die Reaktionsführung, Aufarbeitung und Isolierung der Endprodukte der Formel (VI) erfolgt in üblicher Art und Weise. Die Acylierungsmittel der Formel (VII) sind allgemein bekannt.

Herstellungsbeispiele

Beispiel 1

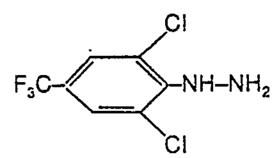

6,2 g (0,025 Mol) 3,4,5-Trichlor-trifluormethylbenzol und 6,25 g (0,125 Mol) Hydrazinhydrat werden in 12 ml Pyridin 48 Stunden bei 115–120 °C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten organischen Phasen werden über Magnesiumsulfat getrocknet, im Vakuum eingeengt und anschließend destilliert.

Man erhält 5,1 g (83% der Theorie) an 2,6-Di-chlor-4-trifluormethylphenylhydrazin vom Schmelzpunkt 56 bis 57 °C mit einem gaschromatographisch bestimmten Gehalt von 90%.

Beispiel 2

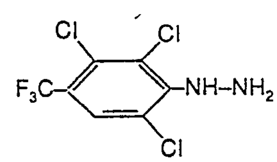

200 g (0,704 Mol) 2,3,4,5-Tetrachlor-trifluormethylbenzol und 240 ml (247,2 g/4,94 Mol) Hydrazinhydrat im 500 ml Dioxan werden 14 Stunden bei 100–105 °C unter Rückfluß erhitzt. Von der abgekühlten zweiphasigen Reaktionsmischung trennt man die schwere (wäßrige) Phase ab und engt die organische Phase im Vakuum zur Trockne ein. Der Rückstand wird in 600 ml Wasser und 100 ml Dichlormethan suspendiert, mit 10%iger wäßriger Natriumhydroxid-Lösung auf pH 10 eingestellt und

langsam auf 30 °C bis 35 °C erwärmt, wobei sich aus der trüben Suspension zwei klare Phasen bilden. Man läßt auf Raumtemperatur abkühlen, trennt die organische Phase ab, wäscht sie mit 200 ml konzentrierter wäßriger Natriumchlorid-Lösung, trocknet über Magnesiumsulfat und entfernt das Lösungsmittel im Vakuum. Das Rohprodukt wird in 370 ml siedendem Hexan 3 bis 4 Stunden gerührt, danach 15 Stunden bei 0 °C bis 5 °C gekühlt, kalt abgesaugt und 2 bis 3 Stunden bei 50 °C im Vakuum getrocknet.

Man erhält 146 g (71,2% der Theorie) an 2,3,6-Trichlor-4-trifluormethyl-phenylhydrazin vom Schmelzpunkt 67 bis 70 °C mit einem gaschromatographisch bestimmten Gehalt von 96%.

Beispiel 3

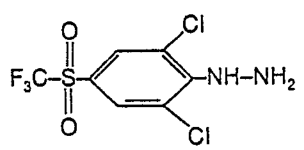

Zu 41 g (0,82 Mol) Hydrazinhydrat in 136 ml Dioxan tropft man bei 20 °C bis 40 °C unter Rühren 85 g (0,27 Mol) 1,2,3-Trichlor-5-trifluormethylsulfonylbenzol in 68 ml Dioxan. Nach beendeter Zugabe rührt man 2 Stunden bei 100–105 °C unter Rückfluß. Nach dem Abkühlen versetzt man die Reaktionsmischung mit Wasser und saugt den entstandenen Niederschlag ab. Nach dem Trocknen kristallisiert man aus Toluol um.

Man erhält 68 g (81% der Theorie) an 2,6-Dichlor-4-trifluormethylsulfonyl-phenylhydrazin vom Schmelzpunkt 135 bis 137 °C.

In entsprechender Weise und gemäß den allgemeinen Herstellungsangaben kann man die folgenden substituierten Phenylhydrazine der Formel (I) erhalten:

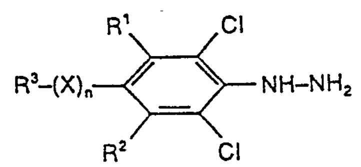

(I)

Tabelle 1

| Bsp. Nr. | $R^1$ | $R^2$ | X | $R^3$ | n | Schmelzpunkt/°C |
|---|---|---|---|---|---|---|
| 4 | H | H | S | $CF_3$ | 1 | 60–62 |
| 5 | Cl | Cl | S | $CF_3$ | 1 | |
| 6 | Cl | H | $SO_2$ | $CF_3$ | 1 | |
| 7 | H | H | $SO_2$ | $CCl_2F$ | 1 | |
| 8 | H | H | $SO_2$ | $CClF_2$ | 1 | |
| 9 | H | H | SO | $CF_3$ | 1 | |

Herstellung der Ausgangsverbindung

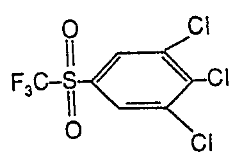

Eine Mischung aus 1123 g (4,6 Mol) 4-Trifluormethylsulfonylchlorbenzol, 11,5 g (0,13 Mol) Eisen(II)sulfid und 11,5 g (0,045 Mol) Iod werden bei 100 °C bis 120 °C mit Chlor versetzt. Die Reaktion wird gaschromatographisch verfolgt und bei 36% Gehalt an gewünschtem Produkt abgebrochen. Die Aufarbeitung erfolgt durch fraktionierte Destillation.

Man erhält 554 g (38,5% der Theorie) an 3,4,5-Trichlortrifluormethylsulfonylbenzol vom Siedepunkt 143 °C bei 22 mbar und vom Schmelzpunkt 102 °C bis 103 °C.

Herstellungsbeispiel für eine herbizid wirksame Verbindung:

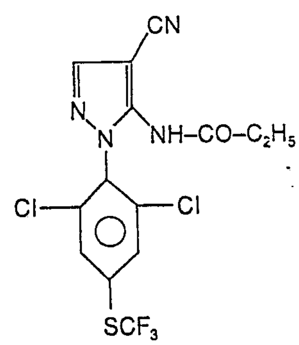

(VI-1)

Eine Suspension von 3,5 g (0,01 Mol)
5-Amino-4-cyano-1-(2,6-dichlor-4-trifluormethylthio-phenyl)-pyrazol
in 30 ml Chloroform werden unter Rühren bei 0 °C zunächst mit 10 ml (0,11 Mol) Propionylchlorid und danach mit 1,8 ml (0,02 Mol) Pyridin in 15 ml Chloroform versetzt. Man erhält eine klare Lösung, die nach beendeter Zugabe weitere 20 Stunden bei Raumtemperatur gerührt wird. Die so erhaltene Lösung wird zur Trockne eingedampft. Zur Aufarbeitung nimmt man in 50 ml Ethanol auf, gibt wäßrigen Ammoniak zu bis zur alkalischen Reaktion, erhitzt 10 Minuten am Rückfluß, entfernt die flüchtigen Bestandteile im Vakuum, nimmt den Rückstand in 100 ml Chloroform auf, wäscht mit Wasser, dann mit 2N wäßriger Salzsäure und wieder mit Wasser, trocknet über Natriumsulfat und entfernt das Lösungsmittel im Vakuum. Man erhält 3,4 g (83% der Theorie) an

**5-Propionylamino-4-cyano-1-(2,6-dichlor-4-trifluormethylthiophenyl)-pyrazol**

vom Schmelzpunkt 153 bis 156 °C.

Herstellung der Ausgangsverbindungen

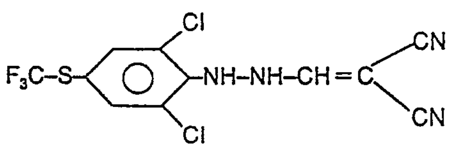

(V-1)

Zu einer Suspension von 13,9 g (0,05 Mol) (2,6-Dichlor-4-trifluormethylthio)-phenylhydrazin und 2,1 g (0,025 Mol) Natriumacetat in 25 ml Eisessig gibt man unter Rühren 6,1 g (0,05 Mol) Ethoxymethylenmalonsäuredinitril. Nach beendeter Zugabe rührt man eine weitere Stunde bei Raumtemperatur und filtriert den so erhaltenen Feststoff ab, welcher nacheinander mit Wasser, wäßriger Natriumhydrogencarbonat-Lösung und wieder mit Wasser gewaschen und anschließend getrocknet wird. Man erhält 15,8 g (89% der Theorie) an

1-(2,2-Dicyanethen-1-yl)-2-(2,6-dichlor-4-trifluormethylthiophenyl)-hydrazin

vom Schmelzpunkt 160 °C.

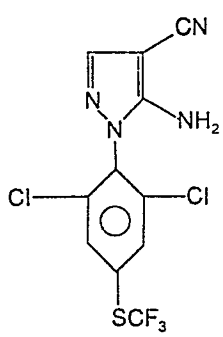

(III-1)

14,1 g (0,04 Mol) 1-(2,2-Dicyanethen-1-yl)-2-(2,6-dichlor-4-trifluormethylthio-phenyl)-hydrazin in 30 ml Ethylenglykolmonoethylether werden 2 Stunden unter Rückfluß erhitzt. Die heiße Lösung wird mit Aktivkohle versetzt, filtriert und mit 60 ml Wasser verdünnt. Der ausgefallene Niederschlag wird abgesaugt und getrocknet. Man erhält 9,8 g (70% der Theorie) an 5-Amino-4-cyano-1-(2,6-dichlor-4-trifluormethylthiophenyl)-pyrazol vom Schmelzpunkt 185 bis 187 °C.

Anwendungsbeispiel

In dem folgenden Anwendungsbeispiel wird die nachstehend aufgeführte Verbindung als Vergleichssubstanz eingesetzt:

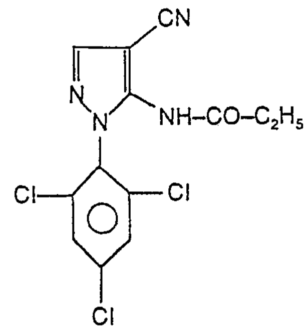

(A)

4-Cyano-5-propionylamino-1-(2,4,6-trichlorphenyl)-pyrazol (bekannt aus DE-OS 3 226 513).

Beispiel A

Pre-emergence-Test

| | |
|---|---|
| Lösungsmittel: | 5 Gew.-Teile Aceton |
| Emulgator: | 1 Gew.-Teil Alkylarylpolyglykolether |

Zur Herstellung einer zweckmäßigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Beispiel zeigt z.B. die Verbindung gemäß Herstellungsbeispiel (VI-1) eine deutliche Überlegenheit in der herbiziden Wirksamkeit ebenso wie in der Nutzpflanzenselektivität im Vergleich zum Stand der Technik; dies gilt insbesondere für Weizen.

**Patentansprüche**

1. Verfahren zur Herstellung von substituierten Phenylhydrazinen der Formel (I)

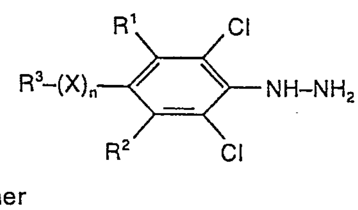

(I)

in welcher

$R^1$ und $R^2$ für Wasserstoff oder Chlor stehen,

$R^3$ für Halogenalkyl steht,

X für Sauerstoff, Schwefel, Sulfinyl oder Sulfonyl steht und

n für eine Zahl 0 oder 1 steht,

dadurch gekennzeichnet, daß man substituierte Chlorbenzole der Formel (II)

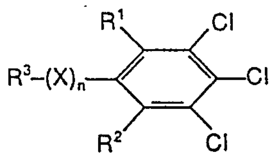

(II)

in welcher

$R^1$ und $R^2$ für Wasserstoff oder Chlor stehen,

$R^3$ für Halogenalkyl steht,

X für Sauerstoff, Schwefel, Sulfinyl oder Sulfonyl steht und

n für eine Zahl 0 oder 1 steht,

mit Hydrazin oder Hydrazinhydrat, gegebenenfalls in Gegenwart eines Verdünnungsmittels und gegebenenfalls bei erhöhtem Druck bei Temperaturen zwischen 60 und 160 °C umsetzt.

2. Verfahren nach Anspruch 1, dadurch gekennzeichnet, daß man die Umsetzung bei Temperaturen zwischen 80 und 140 °C durchführt.

3. Verfahren nach Anspruch 2, dadurch gekennzeichnet, daß man die Umsetzung bei Temperaturen zwischen 100 und 120 °C durchführt.

4. Verfahren nach Anspruch 1, dadurch gekennzeichnet, daß man auf 1 Mol substituiertes Chlorbenzol der Formel (II) 1 bis 10 Mol Hydrazin oder Hydrazinhydrat einsetzt.

5. Verfahren nach Anspruch 4, dadurch gekennzeichnet, daß man auf 1 Mol substituiertes Chlorbenzol der Formel (II) 2 bis 8 Mol Hydrazin oder Hydrazinhydrat einsetzt.

6. Verfahren nach Anspruch 5, dadurch gekennzeichnet, daß man auf 1 Mol substituiertes Chlorbenzol der Formel (II) 3,0 bis 7,0 Mol Hydrazin oder Hydrazinhydrat einsetzt.

7. Verfahren nach Anspruch 1, dadurch gekennzeichnet, daß man in Gegenwart eines Verdünnungsmittels arbeitet, welches aus der Gruppe der inerten organischen Lösungsmittel, wie beispielsweise Dioxan, n-Propanol oder Pyridin ausgewählt wird.

**Claims**

1. Process for the preparation of substituted phenylhydrazines of the formula (I)

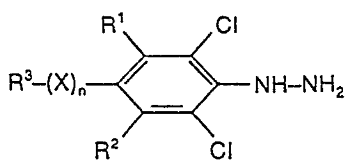

(I)

in which

$R^1$ and $R^2$ represent hydrogen or chlorine,

$R^3$ represents haylogenoalkyl,

X represents oxygen, sulphur, sulphinyl or sulphonyl and

n represents a number 0 or 1,

characterised in that substituted chlorobenzenes of the formula (II)

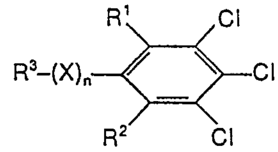

(II)

in which

$R^1$ and $R^2$ represent hydrogen or chlorine,

$R^3$ represents halogenoalkyl,

X represents oxygen, sulphur, sulphinyl or sulphonyl and

n represents a number 0 or 1,

are reacted with hydrazine or hydrazine hydrate, if appropriate in the presence of a diluent and if appropriate at elevated pressure, at temperatures between 60 and 160 °C.

2. Process according to Claim 1, characterised in that the reaction is carried out at temperatures between 80 and 140 °C.

3. Process according to Claim 2, characterised in that the reaction is carried out at temperatures between 100 and 120 °C.

4. Process according to Claim 1, characterised in that 1 to 10 moles of hydrazine or hydrazine hydrate are employed per mole of substituted chlorobenzene of the formula (II).

5. Process according to Claim 4, characterised in that 2 to 8 moles of hydrazine or hydrazine hydrate are employed per mole of substituted chlorobenzene of the formula (II).

6. Process according to Claim 5, characterised in that 3.0 to 7.0 moles of hydrazine or hydrazine hydrate are employed per mole of substituted chlorobenzene of the formula (II).

7. Process according to Claim 1, characterised in that it is carried out in the presence of a diluent which is selected from the group of the inert organic solvents, such as, for example, dioxane, n-propanol or pyridine.

**Revendications**

1. Procédé de préparation de phénylhydrazines substituées de formule I

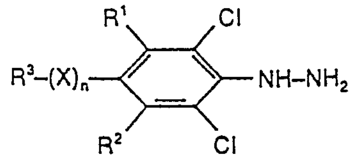

(I)

dans laquelle

$R^1$ et $R^2$ représentent l'hydrogène ou le chlore,

$R^3$ représente un groupe halogénoalkyle,

X représente l'oxygène, le soufre, un groupe sulfinyle ou sulfonyle et

n est égal à 0 ou 1,

caractérisé en ce que l'on fait réagir des chlorobenzènes substitués de formule II

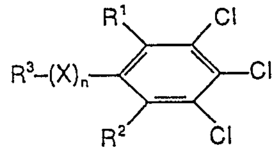

(II)

dans laquelle

$R^1$ et $R^2$ représentent l'hydrogène ou le chlore,

$R^3$ représente un groupe halogénoalkyle,

X représente l'oxygène, le soufre, un groupe sulfinyle ou sulfonyle et

n est égal à 0 ou 1,

avec l'hydrazine ou l'hydrate d'hydrazine, éventuellement en présence d'un diluant et éventuellement sous pression, à des températures de 60 à 160 °C.

2. Procédé selon la revendication 1, caractérisé en ce que l'on effectue la réaction à des températures de 80 à 140 °C.

3. Procédé selon la revendication 2, caractérisé en ce que l'on effectue la réaction à des températures de 100 à 120 °C.

4. Procédé selon la revendication 1, caractérisé en ce que l'on utilise de 1 à 10 moles d'hydrazine ou d'hydrate d'hydrazine pour 1 mole du chlorobenzène substitué de formule II.

5. Procédé selon la revendication 4, caractérisé en ce que l'on utilise de 2 à 8 moles d'hydrazine ou d'hydrate d'hydrazine par mole du chlorobenzène substitué de formule II.

6. Procédé selon la revendication 5, caractérisé en ce que l'on utilise de 3,0 à 7,0 moles d'hydrazine ou d'hydrate d'hydrazine par mole du chlorobenzène substitué de formule II.

7. Procédé selon la revendication 1, caractérisé en ce que l'on opère en présence d'un diluant choisi dans le groupe des solvants organiques inertes, par exemple le dioxanne, le n-propanol ou la pyridine.